# DEMANDE DE BREVET EUROPEEN

(11) **EP 1 166 771 A1**
(43) Date de publication de la demande: **02.01.2002**
(21) Numéro de dépôt: 01401595.2
(22) Date de dépôt: 18.06.2001
(51) Int. Cl.: A61K 7/48

(54) **Nouveaux latex inverses autoinversibles sur des esters d'acides gras, compositions cosmétiques, dermocosmétiques, dermopharmaceutiques ou pharmaceutiques en comportant**

(30) Priorité: 28.06.2000 FR 0008303
(71) Demandeur: SOCIETE D'EXPLOITATION DE PRODUITS POUR LES INDUSTRIES CHIMIQUES, S.E.P.P.I.C., 75321 Paris Cédex 07 (FR)
(72) Inventeur: Tabacchi, Guy, 81100 Castres (FR); Amalric, Chantal, 81700 Blan (FR); Mallo, Paul, 78400 Chatou (FR); Boiteux, Jean-Pierre, 81710 Saix (FR); Michel, Nelly, 94700 Maisons Alfort (FR)
(74) Mandataire: Conan, Philippe Claude

(57) **Abrégé**

Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, caractérisé en ce que ledit polyélectrolyte est, soit un homopolymère à base d'un monomère possédant ou bien une fonction acide fort partiellement ou totalement salifiée, ou bien une fonction acide faible partiellement ou totalement salifiée, soit un copolymère à base d'au moins un monomère possédant une fonction acide fort, copolymérisé ou bien avec au moins un monomère possédant une fonction acide faible, ou bien avec au moins un monomère neutre, soit un copolymère à base d'au moins un monomère possédant une fonction acide faible, copolymérisé avec au moins un monomère neutre et caractérisé en ce que le solvant constitutif de la phase huile est choisi parmi les esters d'acides gras. Composition cosmétique, dermocosmétique, dermopharmaceutique ou pharmaceutique en comportant.

## Description

La présente demande de brevet concerne des latex inverses eau dans huile, leur procédé de préparation et leur application en tant qu'épaississants et/ou émulsionnants pour des produits de soins de la peau et des cheveux ou pour la fabrication de préparations cosmétiques, dermocosmétiques, dermopharmaceutiques ou pharmaceutiques.

Des polymères épaississants synthétiques, se présentant sous forme de latex inverses, sont décrits comme pouvant être utilisés dans la fabrication de compositions topiques, dans les demandes de brevet français publiées sous les numéros 2721511, 2733805, 2774688, 2774996 et 2782086 ainsi que dans la demande de brevet européen publiée sous le numéro EP 0 503 853.

Cependant, certains d'entre eux engendrent parfois des réactions d'intolérance de certaines peaux sensibles.

C'est pourquoi la demanderesse s'est intéressée à la recherche de nouvelles émulsions de polymères, qui soient mieux tolérées par la peau que celles de l'état de la technique.

L'invention a pour objet une composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, caractérisé en ce que ledit polyélectrolyte est, soit un homopolymère à base d'un monomère possédant ou bien une fonction acide fort partiellement ou totalement salifiée, ou bien une fonction acide faible partiellement ou totalement salifiée, soit un copolymère à base d'au moins un monomère possèdant une fonction acide fort, copolymérisé ou bien avec au moins un monomère possédant une fonction acide faible, ou bien avec au moins un monomère neutre, soit un copolymère à base d'au moins un monomère possédant une fonction acide faible, copolymérisé avec au moins un monomère neutre et caractérisé en ce que le solvant constitutif de la phase huile est choisi parmi les esters d'acides gras.

Par ester d'acide gras, on entend dans le cadre de la présente invention, un composé de formule (I) :

R₁-(C=O)-O-[[CH₂-CH[O-[C(=O)]ₘ-R₂]-CH₂-O]ₙ-[C(=O)]ₚ]_{q}-R₃ (I)

dans laquelle :
R₁ représente une chaîne hydrocarbonée saturée ou insaturée, linéaire ou ramifiée comportant de 7 à 30 atomes de carbone,
R₂ représente indépendamment de R₁, un atome d'hydrogène, une chaîne hydrocarbonée saturée ou insaturée linéaire ou ramifiée comportant de 7 à 30 atomes de carbone,
R₃ représente indépendamment de R₁ ou de R₂, un atome d'hydrogène ou une chaîne hydrocarbonée saturée ou insaturée, linéaire ou ramifiée comportant de 1 à 30 atomes de carbone,
m, n, p et q sont indépendamment l'un de l'autre égaux à 0 ou à 1, étant entendu que lorsque R₃ représente un atome d'hydrogène, q est différent de 0.

Dans la formule (I) telle que définie précédemment, R₁, R₂ et R₃ représentent notamment, indépendamment les uns des autres, un radical choisi parmi les radicaux heptyle, octyle, nonyle, décyle, undécyle, dodécyle, tridécyle, tétradécyle, pentadécyle, hexadécyle, heptadécyle, octadécyle, nonadécyle, eicosyle, heneicosyle, docosyle, heptadécènyle, eicosènyle, heneicosènyle, docosènyle, heptadécadiènyle ou décènyle ; le groupe R₁-C(=O)- représente plus particulièrement l'un des radicaux octanoyle (caprylyle), décanoyle, undécylènoyle, dodécanoyle (lauroyle), tétradécanoyle (myristyle), hexadécanoyle (palmitoyle), octadécanoyle (stéaryle), eicosanoyle (arachidoyle), docosanoyle (béhènoyle), 8-octadécènoyle (oléyle), éicosènoyle (gadoleoyle), 13-docosènoyle (érucyle), 9,12-octadécadiènoyle (linoléoyle) ou 9,12,15-octadécatriénoyle (linolénoyle).

Selon un premier aspect particulier de la présente invention, le solvant constitutif de la phase huile du latex inverse, est un composé de formule (la) :

R₁-(C=O)-O-CH₂-CH[O-[C(=O)]ₘ-R₂]-CH₂-O-[C(=O)]ₚ-R₃ (Ia)

correspondant à la formule (I) telle définie précédemment dans laquelle q et n sont égaux à 1, ou un mélange de composés de formule (la).

Lorsque le solvant constitutif de la phase huile du latex inverse, est un composé de formule (la), il s'agit de préférence,
ou bien d'un composé de formule (la₁) :

R₁-(C=O)-O-CH₂-CH(OH)-CH₂-OH (la₁)

correspondant à la formule (la) telle définie précédemment, dans laquelle m et p sont égaux à 0 et R₂ et R₃ représentent un atome d'hydrogène,
ou bien un composé de formule (la₂) :

R₁-(C=O)-O-CH₂-CH(OH)-CH₂-O-C(=O)-R₃ (la₂)

correspondant à la formule (la) telle définie précédemment dans laquelle p est égal à 1, m est égal à 0 et R₂ représente un atome d'hydrogène,
ou bien un composé de formule (la₃) :

R₁-(C=O)-O-CH₂-CH[O-C(=O)-R₂]-CH₂-O-C(=O)-R₃ (la₃)

correspondant à la formule (la) telle définie précédemment dans laquelle m et p sont égaux à 1,
ou bien un mélange de composés de formules (la₁), (la₂) et/ou (la₃).

Comme exemples de composés de formules (la₁), (la₂) ou (la₃), il y a par exemple les triglycérides d'acides gras ou de mélanges d'acides gras tels que le mélange de triglycérides d'acides gras comportant de 6 à 10 atomes de carbone, commercialisé sous le nom SOFTENOL™ 3819, le mélange de triglycérides d'acides gras comportant de 8 à 10 atomes de carbone, commercialisé sous le nom SOFTENOL™ 3108, le mélange de triglycérides d'acides gras comportant de 8 à 18 atomes de carbone, commercialisé sous le nom SOFTENOL™ 3178, le mélange de triglycérides d'acides gras comportant de 12 à 18 atomes de carbone, commercialisé sous le nom SOFTENOL™ 3100, le mélange de triglycérides d'acides gras comportant 7 atomes de carbone commercialisé sous le nom SOFTENOL™ 3107, le mélange de triglycérides d'acides gras comportant 14 atomes de carbone commercialisé sous le nom SOFTENOL™ 3114 ou le mélange de triglycérides d'acides gras comportant 18 atomes de carbone commercialisé sous le nom SOFTENOL™ 3118, le dilaurate de glycérol, le dioléate de glycérol, l'isostéarate de glycérol, le distéarate de glycérol, le monolaurate de glycérol, le monooléate de glycérol, le monoisostéarate de glycérol, le monostéarate de glycérol, ou un mélange de ces composés.

Selon un deuxième aspect particulier de la présente invention, le solvant constitutif de la phase huile du latex inverse, est un composé de formule (lb) :

R₁-(C=O)-O-R₃ (Ib)

correspondant à la formule (I) telle définie précédemment dans laquelle q est égal à 0, ou un mélange de composés de formule (lb).

Comme exemples de composés de formule (lb), il y a par exemple, le palmitate d'octyle.

Selon un troisième aspect particulier de la présente invention, le solvant constitutif de la phase huile du latex inverse, est un mélange d'au moins un composé de formule (lb) et d'au moins un composé de formule (la).

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

Par "agent émulsifiant du type eau dans huile", on désigne des agents émulsifiants possédant une valeur de HLB suffisamment faible pour fournir des émulsions eau dans huile tels que les polymères tensioactifs commercialisés sous le nom de HYPERMER™ ou tels que les esters de sorbitan, comme le monooléate de sorbitan commercialisé par la Société SEPPIC sous le nom de marque MONTANE™ 80 ou l'isostéarate de sorbitan commercialisé par SEPPIC sous le nom de MONTANE™ 70. On peut aussi adjoindre à ces agents émulsifiants, l'oléate de sorbitan éthoxylé avec 5 moles d'oxyde d'éthylène, commercialisé par la société SEPPIC sous le nom de MONTANOX™ 81.

Par "agent émulsifiant du type huile dans eau", on désigne des agents émulsifiants possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène, commercialisé par la société SEPPIC sous le nom de MONTANOX™ 80, l'huile de ricin éthoxylée à 40 moles d'oxyde d'éthylène commercialisée par la société SEPPIC sous le nom de SIMULSOL™ OL50, le laurate de sorbitan éthoxylé à 20 moles d'oxyde d'éthylène commercialisé par la société SEPPIC sous le nom de MONTANOX™ 20 ou l'alcool laurique éthoxylé à 7 moles d'oxyde d'éthylène, commercialisé par la société SEPPIC sou le nom de SIMULSOL™ P7.

Comme agents émulsifiants possédant une valeur de HLB suffisamment élevée pour fournir des émulsions huile dans l'eau, il y a aussi les composés de formule (II) :

R₄-O-[CH(R₅)-CH₂-O]ₙ-(G)ₓ-H (II)

dans laquelle R₄ représente un radical hydrocarboné linéaire ou ramifié, saturé ou insaturé comprenant de 1 à 30 atomes de carbones, R₅ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30.

Par reste d'un saccharide, on désigne pour G, un radical bivalent résultant de l'enlèvement sur une molécule de sucre, d'une part d'un atome d'hydrogène d'un groupe hydroxyle et d'autre part du groupe hydroxyle anomérique. Le terme saccharide désigne notamment le glucose ou dextrose, le fructose, le mannose, le galactose, l'altrose, l'idose, l'arabinose, le xylose, le ribose, le gulose, le lyxose, le maltose, le maltotriose, le lactose, le cellobiose, le dextrane, le talose, l'allose, le raffinose, le lévoglucane, la cellulose ou l'amidon. La structure oligomérique (G)ₓ peut se présenter sous toute forme d'isomérie, qu'il s'agisse d'isomérie optique, d'isomérie géométrique ou d'isomérie de position ; elle peut aussi représenter un mélange d'isomères. Dans la formule (II) telle que définie précédemment, le radical R₄-O-[CH(R₅)-CH₂-O]ₙ- est lié à G, par le carbone anomérique de manière à former une fonction acétal. Le groupe divalent -[CH(R₅)-CH₂-O]ₙ- représente, soit une chaîne composée uniquement de groupes éthoxyle (R₅ = H), soit une chaîne composée uniquement de groupes propoxyle (R₅ = CH₃), soit une chaîne composée à la fois de groupes éthoxyle et de groupes propoxyle. Dans ce dernier cas, les fragments -CH₂-CH₂-O- et -CH(CH₃)-CH₂-O- sont distribués dans ladite chaîne, de façon séquencée ou aléatoire. Le nombre x, qui représente dans la formule (II) le degré moyen de polymérisation du saccharide, est plus particulièrement compris entre 1 et 3, notamment entre 1,05 et 2,5, tout particulièrement entre 1,1 et 2,0 et de préférence, inférieur ou égal à 1,5. Comme agents tensioactifs émulsifiants possédant une valeur de HLB suffisamment élevée pour fournir des émulsions huile dans l'eau, il y a plus particulièrement, les composés de formule (II) telle que définie précédemment dans laquelle G représente le reste du glucose ou le reste du xylose et/ou dans laquelle n est égal à 0, et/ou dans laquelle R₄ représente un radical comportant de 8 à 18 atomes de carbone et tout particulièrement dans laquelle R₄ représente plus particulièrement un radical octyle, décyle, undécyle, dodécyle, tétradécyle ou hexadécyle, lesdits radicaux étant linéaires ou ramifiés.

Comme exemples de produits commerciaux contenant lesdits composés, il y a par exemple :

Le SIMULSOL™ SL8, commercialisé par la société SEPPIC, qui est une solution aqueuse contenant entre environ 35% et 45% en poids d'un mélange d'alkyl polyglycosides consistant en entre 45% en poids et 55% en poids d'un composé de formule (II), dans laquelle G représente le reste du glucose, x est égal à environ 1,45, n est égal à 0 et R₄ représente un radical décyle, et entre 45% en poids et 55% en poids d'un composé de formule (II), dans laquelle G représente le reste du glucose, x est égal à environ 1,45, n est égal à 0 et R₄ représente un radical octyle ;

Le SIMULSOL™ SL10, commercialisé par la société SEPPIC, qui est une solution aqueuse contenant entre environ 40% en poids et 50% en poids d'un mélange d'alkyl polyglycosides, consistant en environ 85% en poids d'un composé de formule (II), dans laquelle G représente le reste du glucose, x est égal à environ 1,45, n est égal à 0 et R₄ représente un radical décyle, environ 7,5% en poids d'un composé de formule (II), dans laquelle G représente le reste du glucose, x est égal à environ 1,45, n est égal à 0 et R₄ représente un radical dodécyle et environ 7,5% en poids d'un composé de formule (II), dans laquelle G représente le reste du glucose, x est égal à environ 1,45, n est égal à 0 et R₄ représente un radical tétradécyle ;

Le SIMULSOL™ SL11, commercialisé par la société SEPPIC, qui est une solution aqueuse contenant entre environ 40% en poids et 50% en poids d'un mélange d'alkyl polyglycosides de formule (II), dans laquelle G représente le reste du glucose, x est égal à environ 1,45, n est égal à 0 et R₄ représente un radical undécyle ; ou

Le SIMULSOL™ SL26, commercialisé par la société SEPPIC, qui est une solution aqueuse contenant entre environ 40% en poids et 55% en poids d'un mélange d'alkyl polyglycosides consistant en environ 70% en poids d'un composé de formule (II), dans laquelle G représente le reste du glucose, x est égal à environ 1,45, n est égal à 0 et R₄ représente un radical dodécyle, environ 25% en poids d'un composé de formule (II), dans laquelle G représente le reste du glucose, x est égal à environ 1,45, n est égal à 0 et R₄ représente un radical tétradécyle et environ 5% en poids d'un composé de formule (II), dans laquelle G représente le reste du glucose, x est égal à environ 1,45, n est égal à 0 et R₄ représente un radical hexadécyle.

La fonction acide fort du monomère en comportant est notamment la fonction acide sulfonique ou la fonction acide phosphonique, partiellement ou totalement salifiée. Ledit monomère peut être par exemple l'acide styrènesulfonique partiellement ou totalement salifié. Il est de préférence l'acide 2-méthyl 2-[(1-oxo 2-propènyl) amino] 1-propanesulfonique partiellement ou totalement salifiée sous forme d'un sel de métal alcalin tel que par exemple le sel de sodium ou le sel de potassium, de sel d'ammonium, d'un sel d'un amino alcool tel que par exemple le sel de monéthanolamine ou d'un sel d'amino acide tel que par exemple le sel de lysine.

La fonction acide faible du monomère en comportant est notamment la fonction acide carboxylique, et de préférence, ledit monomère est choisi parmi l'acide acrylique, l'acide méthacrylique, l'acide itaconique ou l'acide maléique partiellement ou totalement salifié.

Le monomère neutre est notamment choisi parmi l'acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters.

Selon un quatrième aspect particulier de la présente invention, le polyélectrolyte compris dans le latex inverse tel que défini précédemment, est un homopolymère de l'acide acrylique partiellement ou totalement salifié sous forme de sel de sodium ou de sel d'ammonium.

Selon un cinquième aspect particulier de la présente invention, le polyélectrolyte compris dans le latex inverse tel que défini précédemment, est un copolymère d'acide 2-méthyl 2-[(1-oxo 2-propènyl) amino] 1-propanesulfonique partiellement ou totalement salifié (a), et d'acrylate de (2-hydroxy éthyle) (b), dans un rapport molaire (a) / (b) compris entre 30 */* 70 et 90 / 10 et tout particulièrement 50 / 50 et 90 / 10. Le polyélectrolyte est de préférence un copolymère de sel de sodium ou de sel d'ammonium de l'acide 2-méthyl 2-[(1-oxo 2-propènyl) amino] 1-propanesulfonique (a₁) et de 10% à 40% d'acrylate de (2-hydroxy éthyle) (b), dans un rapport molaire (a₁) / (b), compris entre 60 / 40 et 90 / 10.

Selon un sixième aspect particulier de la présente invention, le polyélectrolyte compris dans le latex inverse tel que défini précédemment, est un copolymère de sel de sodium, de sel d'ammonium, de sel de monoéthanolamine ou de sel de lysine de l'acide 2-méthyl 2-[(1-oxo 2-propènyl) amino] 1-propanesulfonique (a₁) et d'acide acrylique partiellement ou totalement salifié sous forme de sel de sodium, de sel d'ammonium, de sel de monoéthanolamine ou de sel de lysine (c₁), dans un rapport molaire (a₁) / (c₁), compris entre 30 / 70 et 90/10 et tout particulièrement entre 30 / 70 et 45 / 55.

Selon un septième aspect particulier de la présente invention, le polyélectrolyte compris dans le latex inverse tel que défini précédemment, est un copolymère de sel de sodium ou de sel d'ammonium de l'acide 2-méthyl 2-[(1-oxo 2-propènyl) amino] 1-propanesulfonique (a₂) et d'acrylamide (d), dans un rapport molaire (a₂) / (d), compris entre 50 / 50 et 30/70.

L'invention a plus particulièrement pour objet une composition telle que définie précédemment, caractérisée en ce que le polyélectrolyte est réticulé et/ou branché avec un composé diéthylénique ou polyéthylénique dans la proportion molaire exprimée par rapportaux monomères mis en oeuvre, de 0,005% à 1%, plus particulièrement de 0,01 % à 0,5 % et tout particulièrement de 0,1 % à 0,25 %. L'agent de réticulation et/ou l'agent de ramification est choisi parmi l'acide diallyoxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène-bis(acrylamide), le triallylamine ou un mélange de ces composés.

Le latex inverse tel que défini ci-dessus, contient généralement de 4% à 10% en poids, d'agents émulsifiants. Généralement de 20% à 50% et plus particulièrement de 25% à 40% du poids total des émulsifiants sont du type eau dans huile et de 80% à 50% et plus particulièrement de 75 à 60% sont du type huile dans eau.

Sa phase huile représente de 15% à 40%, et de préférence de 20% à 25%, de son poids total. Ce latex peut en outre contenir un ou plusieurs additifs choisis notamment parmi les agents complexants, les agents de transfert ou les agents limiteurs de chaînes.

L'invention a aussi pour objet, une composition cosmétique, dermocosmétique, dermopharmaceutique ou pharmaceutique, caractérisée en ce qu'elle comprend comme composé épaississant, au moins un latex inverse tel que défini précédemment.

La composition cosmétique, dermocosmétique, dermopharmaceutique ou pharmaceutique définie ci-desssus comprend généralement de 0,1 % à 10 % et plus particulièrement entre 0,5% et 5% en poids dudit latex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex inverse, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains moussants, les baumes, les shampooings ou les après shampooings. Il peut aussi être utilisé en combinaison avec lesdits SEPIGEL. Il est notamment compatible avec les concentrés décrits et revendiqués dans les publications internationales WO 92/06778, WO 95/04592, W095/13863 ou FR 2734 496 ou avec les agents tensioactifs décrits dans WO 93/08204.

Il est particulièrement compatible avec le MONTANOV™ 68, le MONTANOV™ 82, le MONTANOV™ 202 le MONTANOV™ WO18, le MONTANOV™S ou le SEPIPERL™ N. Il peut également être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024 ou encore en association avec des esters d'acides gras et de sucre, pour former des compositions pour le traitement du cheveux ou de la peau telles que celles décrites dans EP 0 603 019. ou encore dans les shampooings ou après shampooings tels que décrits et revendiqués dans WO 92/21316 ou enfin en association avec un homo polymère anionique tels que le CARBOPOL™ pour former des produits de traitement des cheveux comme ceux décrits dans DE 195 23596. Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249, EP 0576188 ou dans WO 93/07902. Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611. Il est aussi compatible avec les acides glycoliques, avec l'acide lactique, avec l'acide salicylique les rétinoïdes, le phénoxy éthanol, les sucres, le glycéraldéhyde, les xanthanes, les acides de fruit, et les divers polyols utilisés dans la fabrication de formulations cosmétiques.

L'invention a donc aussi pour objet, l'utilisation d'un latex inverse tel que défini précédemment, pour préparer une composition cosmétique, dermocosmétique, dermopharmaceutique ou pharmaceutique.

Les exemples qui suivent ont pour but d'illustrer la présente invention sans toutefois la limiter. Ils montrent que les nouveaux latex inverses n'irritent pas la peau et que leurs propriétés physiques permettent leur utilisation dans la préparation de compositions cosmétiques, dermopharmaceutiques ou pharmaceutiques plus particulièrement destinées au traitement des peaux sensibles.

### A) Exemples de préparations de compositions selon l'invention

### Exemple 1 : Latex inverse d'un copolymère AMPS (sel de Na) / acide acrylique (sel de Na), réticulé au méthylène bis(acrylamide), dans le palmitate d'octyle (Composition 1).

### a) - On charge dans un réacteur sous agitation :

- 2,88 kg d'acide acrylique glacial,
- 22,05 kg d'une solution commerciale à 55% de 2-méthyl 2-[(1-oxo 2-propènyl) amino] 1-propanesulfonate de sodium,
- 1,66 kg d'une solution aqueuse à 48% en poids d'hydroxyde de sodium,
- 0,022 kg de diéthylènetriaminepentacétate de sodium,
- 0,006 kg de méthylène bis(acrylamide).

Le pH de cette solution aqueuse est égal à 4,9 ; on rajoute 7, 42 kg d'eau.

### b) - On prépare une phase organique en mélangeant :

- 5,28 kg de ISOPAR™ G,
- 4,875 kg de palmitate d'octyle,
- 1,416 kg de MONTANE™ 80 VG,
- 0,377 kg de MONTANOX™ 81 VG et
- 0,0078 kg d'azo bis(isobutyronitrile).

### c) - La phase aqueuse est introduite progressivement dans la phase organique et l'ensemble est agité fortement au moyen d'un agitateur SILVERSON™.

L'émulsion obtenue est alors transférée dans un réacteur de polymérisation, soumise à un barbotage d'azote puis refroidit à environ 5-6°C. On ajoute alors 250 ml d'une solution contenant 0,635% en poids d'hydroperoxyde de cumène dans le l'ISOPAR™G, puis, après homogénéisation de la solution, une solution aqueuse de métabisulfite de sodium (0,2% en poids dans l'eau) à raison de 0,2 ml / minute pendant environ 60 minutes et en laissant monter la température jusqu'à la température de polymérisation. On maintient alors le milieu réactionnel pendant environ 90 minutes à cette température, à l'issu desquelles le mélange obtenu est chauffé sous vide partiel pour éliminer l'ISOPAR™G et environ 10 kg d'eau. Le mélange résultant est refroidi jusqu'à 35° environ. On introduit lentement 2,53 kg d'oléate de sorbitan éthoxylé à 20 moles d'oxyde d'éthylène (MONTANOX™ 80) et on obtient l'émulsion eau dans huile désirée.

### Evaluation des propriétés

Viscosité dans l'eau à 3% du latex (Brookfield RVT Mobile 6, vitesse 5) :
η = 50 200 mPa.s
Viscosité à 3% du latex dans de l'eau salée à 0,1% de NaCI (Brookfield RVT Mobile 6, vitesse 5) : η = 25800 mPa.s.

### Exemple 2 : Latex inverse d'un copolymère AMPS (sel de Na) / acide acrylique (sel de Na), réticulé au méthylène bis(acrylamide), dans un mélange dr triglycéride d'acides gras comportant de 8 atomes de carbone (Composition 2)

### a) - On charge dans un bécher sous agitation :

- 55,86 g d'acide acrylique glacial,
- 428,25 g d'une solution commerciale à 55% de 2-méthyl 2-[(1-oxo 2-propènyl) amino] 1-propanesulfonate de sodium,
- 32,34 g d'une solution aqueuse à 48% en poids d'hydroxyde de sodium,
- 0,46 g de diéthylènetriaminepentacétate de sodium,
- 0,106 g de méthylène bis(acrylamide),
- Le pH de cette solution aqueuse est ajustée à 4,9 et on rajoute de l'eau permutée de manière à porter la masse de la phase aqueuse à 625,61 g.

### b) - On prépare une phase organique en mélangeant :

- 57,1 g d'ISOPAR™ G,
- 228,25 g de triglycéride d'acides gras en C₈ - C₁₀ commercialisé en France, par la société STEARINERIE DUBOIS & Fils, sous le nom Triglycérides C₈C₁₀ 5545.
- 8,83 g d'HYPERMER™ B246 (copolymère octadécène / anhydride maléïque commercialisé par lasociété CHEVRON Chemicals),
- 1,47 g de résine PA18 et
- 0,26 g d'azo bis(isobutyronitrile).
**c)** - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 5 ml d'une solution contenant 0,384% en poids d'hydroperoxyde de cumène dans l'ISOPAR™ G, puis après homogénéisation de la solution, une solution aqueuse de métabisulfite de sodium (0,1% dans l'eau) pendant environ 60 minutes et en laissant monter la température jusqu'à la température de polymérisation. On maintient alors le milieu réactionnel pendant environ 90 minutes à cette température, à l'issu desquelles le mélange obtenu est chauffé sous vide partiel pour éliminer l'ISOPAR™G et environ 10 kg d'eau. Le mélange obtenu est refroidi jusqu'à 35° environ. On introduit lentement 10,1 g d'huile de ricin éthoxylée à 40 moles d'oxyde d'éthylène (SIMULSOL™ OL50) et on obtient l'émulsion eau dans huile désirée.

### Evaluation des propriétés

Viscosité dans l'eau à 3% du latex (Brookfield RVT Mobile 6, vitesse 5) :
η = 106 000 mPa.s
Viscosité à 3% du latex dans de l'eau salée (NaCl 0,1 %) (Brookfield RVT Mobile 3, vitesse 5) : η = 37 000 mPa.s.

### B Propriétés des compositions selon l'invention

### a) stabilité à la température

On a préparé un gel crème comprenant 3% de composition 1 et 20% de cétéaryl octanoate et mesuré la viscosité Les résultats sont les suivants:

| | Viscosité Brookfield LVT 6rpm (en mPa.s) | |
|---|---|---|
| | A température ambiante | A 50°C |
| Après 1 jour | 100 000 | 100 000 |
| Après 7 jours | 100 000 | 100 000 |
| Après 1 mois | 100 000 | 100 000 |

### b) Influence du rayonnement UV sur la stabilité

On constate que les gels préparés avec les compositions 1 ou 2 sont très stables eu rayonnement UV; leur viscosité n'ayant pas varié après 14 jours d'exposition.

### c) Influence du pH sur la viscosité

La viscosité des gels préparés avec les compositions 1 ou 2 sont très stables au pH dans l'intervalle pH = 4 à pH = 8

### d) Etude comparative de la tolérance

La tolérance locale épicutanée d'une série de gels-crèmes, contenant 3 % et 5 % en poids d'une des compositions 1 et 2 préparées comme décrit ci-dessus, a été déterminée et comparée à celle observée avec un latex inverse d'un copolymère AMPS/acrylate de sodium réticulé au méthylène bis(acrylamide), dans l'isohexadécane (Composition A), selon le protocole suivant :

On applique la composition à tester, à une surface d'environ 50 mm² de la région sous-scapulaire gauche, de la peau du dos de 38 volontaires sains, parmi lesquels 19 ont une peau de type "peau japonaise" (PJ) et 19 une peau de type "peau caucasienne" (PC). Le contact est maintenu pendant 48 heures sous timbre occlusif.

Cette application est aussi réalisée dans les mêmes conditions avec un timbre seul (sans composition) en tant que témoin négatif.

L'observation clinique de la surface de peau ainsi traitée est réalisée 30 minutes puis 24 heures après la dépose desdits timbres. Ces observations sont faites par comparaison à la surface non traitée témoin négatif.

La quantification de l'irritation cutanée, selon une échelle numérique allant de 0 à 4 (0 : pas d'effet ; 1 : effet très léger ; 2 : effet net ; 3 et 4: effet modéré à sévère selon les réactions), est réalisée pour chacune des réactions éventuellement observées à savoir : érythème, oedème, vésicules, sécheresse de la peau, rugosité de la peau et la réflectivité de la peau.

Les indices de tolérance cutanée (IC), consignés dans le tableau suivant, expriment la moyenne de la somme des effets quantifiés relevés sur chaque volontaire :
IC = 0 signifie qu'aucune irritation n'a été observée,
IC = 0,5 signifie que le produit est statistiquement bien toléré,
IC > 0,5 signifie que le produit engendre une intolérance. (Composition A).

| | Indice de tolérance cutanée de gels à 5% | |
|---|---|---|
| | PJ | PC |
| composition 1 | 0,05 | 0,0 |
| composition 2 | 0,05 | 0,0 |
| composition A | 0,95 | 0,47 |

Ces résultats montrent que de façon inattendue, le palmitate d'octyle et le triglycéride d'acides gras de la composition préparée à l'exemple 2, pontentialisent la tolérance cutanée du polymère du latex inverse.

### C) Exemples de formulations préparées avec les compositions selon l'inver tion

### Exemple 3 : Crème de soin

| | |
|---|---|
| Cyclométhicone | 10% |
| Composition 1 | 0,8% |
| MONTANOV™ 68 | 2% |
| alcool stéarylique | 1% |
| alcool stéarique | 0,5% |
| conservateur | 0,65% |
| Lysine | 0,025% |
| EDTA (sel disodique) | 0,05% |
| Gomme de xanthane | 0,2% |
| Glycérine | 3 % |
| Eau | q.s.p. 100% |

### Exemple 4 : Baume après-rasage

| FORMULE | | |
|---|---|---|
| A | Composition 1 | 1,5% |
| | Eau | q.s.p 100% |
| B | MICROPEARL™ M 100 | 5,0% |
| | SEPICIDE™ CI | 0,50% |
| | Parfum | 0,20% |
| | éthanol 95° | 10,0% |

| MODE OPERATOIRE | | |
|---|---|---|
| Ajouter B dans A. | | |

### Exemple 5 : Emulsion satinée pour le corps

| FORMULE | | |
|---|---|---|
| A | SIMULSOL™ 165 | 5,0% |
| | LANOL™ 1688 | 8,50% |
| | beurre de Karité | 2% |
| | huile de paraffine | 6,5% |
| | LANOL™ 14M | 3% |
| | LANOL™ S | 0,6% |
| B | eau | 66,2% |
| C | MICROPEARL™ M 100 | 5% |
| D | Composition 2 | 3% |
| E | SEPICIDE™ CI | 0,3% |
| | SEPICIDE™ HB | 0,5% |
| | MONTEINE™ CA | 1% |
| | Parfum | 0,20% |
| | acétate de vitamine E | 0,20% |
| | Sodium pyrrolidinonecarboxylate | 1% |

| MODE OPERATOIRE | | |
|---|---|---|
| Ajouter C dans B, émulsionner B dans A à 70°C, puis ajouter D à 60°C puis E à 30°C. | | |

### Exemple 6 : Crème H/E

| FORMULE | | |
|---|---|---|
| A | SIMULSOL™ 165 | 5,0% |
| | LANOL™ 1688 | 20,0% |
| | LANOL™ P | 1,0% |
| B | eau | q.s. 100% |
| C | Composition 2 | 2,50% |
| D | SEPICIDE™ CI | 0,20% |
| | SEPICIDE™ HB | 0,30% |

| MODE OPERATOIRE | | |
|---|---|---|
| Introduire B dans A vers 75°C ; ajouter C vers 60°C, puis D vers 45°C. | | |

### Exemple 7 : gel solaire non gras

| FORMULE | | |
|---|---|---|
| A | Composition 2 | 3,00% |
| | Eau | 30% |
| B | SEPICIDE™ CI | 0,20% |
| | SEPICIDE™ HB | 0,30% |
| | Parfum | 0,10% |
| C | colorant | q.s.p |
| | eau | 30% |
| D | MICROPEARL™ M 100 | 3,00% |
| | Eau | q.s.p 100% |
| E | huile de silicone | 2,0% |
| | PARSOL™ MCX | 5,00% |

| MODE OPERATOIRE | | |
|---|---|---|
| Introduire B dans A; ajouter C, puis D, puis E. | | |

### Exemple 8 : Lait solaire

| FORMULE | | |
|---|---|---|
| A | MONTANOV™ S | 3,0% |
| | Huile de sésame | 5,0% |
| | PARSOL™ MCX | 5,0% |
| | Carraghénane λ | 0,10% |
| B | eau | q.s.p.100% |
| C | Composition 1 | 0,80% |
| D | Parfum | q.s. |
| | Conservateur | q.s. |

| MODE OPERATOIRE | | |
|---|---|---|
| Emulsionner B dans A à 75°C puis ajouter C vers 60°C, puis D vers 30°C et ajuster le pH si nécessaire | | |

### Exemple 9 : Gel de massage

| FORMULE | | |
|---|---|---|
| A | Composition 2 | 3,5% |
| | Eau | 20,0% |
| B | colorant | 2 gouttes/100g |
| | Eau | q. s. |
| C | alcool | 10% |
| | Menthol | 0,10% |
| D | huile de silicone | 5,0% |

| MODE OPERATOIRE | | |
|---|---|---|
| Ajouter B dans A, puis ajouter au mélange, C puis D | | |

### Exemple 10 : Fond de teint hydratant et matifiant

| FORMULE | | |
|---|---|---|
| A | eau | 20,0% |
| | Butylène glycol | 4,0% |
| | PEG-400 | 4,0% |
| | PECOSIL™ PS100 | 1,0% |
| | NaOH | q.s. pH = 9 |
| | Dioxyde de titane | 7,0% |
| | Talc | 2,0% |
| | Oxyde de fer jaune | 0,8% |
| | Oxyde de fer rouge | 0,3% |
| | Oxyde de fer noir | 0,05% |
| B | LANOL™ 99 | 8% |
| | Caprylic capric triglycéride | 8% |
| | MONTANOV™ 202 | 5,00 % |
| C | eau | q.s.p. 100% |
| | MICROPEARL™ M305 | 2,0% |
| | EDTA tétrasodé | 0,05% |
| D | Cyclométhicone | 4,0% |
| | Gomme de Xanthanse | 0,2% |
| | composition 1 | 0,8% |
| E | SEPICIDE™ HB | 0,5% |
| | SEPICIDE CI | 0,3% |
| | Parfum | 0,2% |

| MODE OPERATOIRE | | |
|---|---|---|
| préparer à 80°C, les mélanges B + D et A + C, puis mélanger et émulsionner l'ensemble. | | |

### Exemple 11 : Gel coup d'éclat

| FORMULE | | |
|---|---|---|
| A | Composition 1 | 4% |
| | Eau | 30% |
| B | ELASTINE HPM | 5,0% |
| C | MICROPEARL™ M 100 | 3% |
| | Eau | 5% |
| D | SEPICIDE™ CI | 0,2% |
| | SEPICIDE™ HB | 0,3% |
| | Parfum | 0,06% |
| Sodium pyrrolidinonecarboxylate 50% | | 1% |
| Eau | | q. s. p. 100% |

| MODE OPERATOIRE | | |
|---|---|---|
| Préparer A ; additionner B, puis C, puis D. | | |

### Exemple 12 : Lait corporel

| FORMULE | |
|---|---|
| MONTANOV™ S | 3,5% |
| LANOL™ 37T | 8,0% |
| SOLAGUM™ L | 0,05% |
| Eau | q.s.p.100% |
| Benzophénone | 2,0% |
| diméthicone 350cPs | 0,05% |
| Composition 2 | 0,8% |
| conservateur | 0,2% |
| parfum | 0,4% |

### Exemple 13 : Emulsion démaquillante à l'huile d'amandes douces

| FORMULE | |
|---|---|
| MONTANOV™ 68 | 5% |
| huile d'amandes douces | 5% |
| eau | q.s.p.100% |
| Composition 1 | 0,3% |
| glycérine | 5% |
| conservateur | 0,2% |
| parfum | 0,3% |

### Exemple 14 : Crème hydratante pour peaux grasses

| FORMULE | |
|---|---|
| MONTANOV™ 68 | 5% |
| Cétylstéaryloctanoate | 8% |
| octyl palmitate | 2% |
| eau | q.s.p.100% |
| Composition 2 | 0,6% |
| MICROPEARL™ M100 | 3,0% |
| Mucopolysaccharides | 5% |
| SEPICIDE™ HB | 0,8% |
| Parfum | 0,3% |

### Exemple 15 : Baume après-rasage apaisant sans alcool

| FORMULE | | |
|---|---|---|
| A | LIPACIDE™ PVB | 1,0% |
| | LANOL™ 99 | 2,0% |
| | Huile d'amandes douces | 0,5% |
| B | Composition 1 | 3,5% |
| C | eau | q.s.p.100% |
| D | parfum | 0,4% |
| | SEPICIDE™ HB | 0,4% |
| | SEPICIDE™ CI | 0,2% |

### Exemple 16 : Crème aux AHA pour peaux sensibles

| FORMULE | |
|---|---|
| mélange de lauryl aminoacides | 0,1% à 5% |
| aspartate de magnésium et de potassium | 0,002% à 0,5% |
| LANOL™ 99 | 2% |
| MONTANOV™ 68 | 5,0% |
| Eau | q.s.p.100% |
| Composition 2 | 1,50% |
| acide gluconique | 1,50% |
| tri éthylamine | 0,9% |
| SEPICIDE™ HB | 0,3% |
| SEPICIDE™ CI | 0,2% |
| Parfum | 0,4% |

### Exemple 17 : Soin apaisant après soleil

| FORMULE | |
|---|---|
| mélange de lauryl aminoacides | 0,1% à 5% |
| aspartate de magnésium et de potassium | 0,002% à 0,5% |
| LANOL™ 99 | 10,0% |
| Eau | q.s.p.100% |
| Composition 1 | 2,50% |
| SEPICIDE™ HB | 0,3% |
| SEPICIDE™ CI | 0,2% |
| Parfum | 0,4% |
| Colorant | 0,03% |

### Exemple 18 : Lait démaquillant

| FORMULE | |
|---|---|
| SEPIPERL™ N | 3% |
| PRIMOL™ 352 | 8,0% |
| huile d'amandes douces | 2 % |
| eau | q.s.p.100% |
| Composition 2 | 0,8% |
| conservateur | 0,2% |

### Exemple 19 : Emulsion fluide à pH alcalin

| | |
|---|---|
| MARCOL™ 82 | 5,0% |
| NaOH | 10,0% |
| Eau | q.s.p.100% |
| Composition 1 | 1,5% |

### Exemple 20 : Fond de teint fluide

| FORMULE | |
|---|---|
| SIMULSOL™ 165 | 5,0% |
| LANOL™ 84D | 8,0% |
| LANOL™ 99 | 5,0% |
| Eau | q.s.p.100% |
| pigments et charges minérales | 10,0% |
| Composition 1 | 1,2% |
| conservateur | 0,2% |
| parfum | 0,4% |

### Exemple 21 : Lait solaire

| FORMULE | |
|---|---|
| SEPIPERL™ N | 3,5% |
| LANOL™ 37T | 10,0% |
| PARSOL™ MCX | 5,0% |
| EUSOLEX™ 4360 | 2,0% |
| Eau | q.s.p.100% |
| Composition 1 | 1,8% |
| conservateur | 0,2% |
| parfum | 0,4% |

### Exemple 22 : Gel contour des yeux

| FORMULE | |
|---|---|
| Composition 1 | 2,0% |
| Parfum | 0,06% |
| Sodium pyrrolidinonecarboxylate | 0,2% |
| DOW CORNING™ 245 Fluid | 2,0% |
| Eau | q. s. p. 100% |

### Exemple 23 : Composition de soin non rincée

| FORMULE | |
|---|---|
| Composition 1 | 1,5% |
| Parfum | q. s |
| Conservateur | q. s. |
| DOW CORNING™ X2 8360 | 5,0% |
| DOW CORNING™ Q2 1401 | 15,0% |
| Eau | q.s.p. 100% |

### Exemple 24 : Gel amincissant

| | |
|---|---|
| Composition 1 | 5 % |
| Ethanol | 30 % |
| Menthol | 0,1 % |
| Caféine | 2,5 % |
| extrait de ruscus | 2 % |
| extrait de lierre | 2 % |
| SEPICIDE™ HP | 1 % |
| Eau | q. s. p. 100 % |

### Exemple 25 : Gel crème teinté ultra naturel

| FORMULE | | |
|---|---|---|
| A | eau | 10,0% |
| | Butylène glycol | 4,0% |
| | PEG-400 | 4,0% |
| | PECOSIL™ PS100 | 1,5% |
| | NaOH | q.s. pH = 7 |
| | Dioxyde de titane | 2,0% |
| | Oxyde de fer jaune | 0,8% |
| | Oxyde de fer rouge | 0,3% |
| | Oxyde de fer noir | 0,05% |
| B | LANOL™ 99 | 4,0% |
| | Caprylic capric triglycéride | 4,0% |
| | SEPIFEEL™ ONE | 1,0% |
| | Composition 1 | 3,0% |
| C | eau | q.s.p. 100% |
| | MICROPEARL™ M305 | 2,0% |
| | EDTA tétrasodé | 0,05% |
| | Cyclométhicone | 4,0% |
| D | SEPICIDE™ HB | 0,5% |
| | SEPICIDE CI | 03% |
| | Parfum | 0,2% |

| MODE OPERATOIRE | | |
|---|---|---|
| préparer le mélange B + C puis ajouter A puis D. | | |

### Exemple 26 : Soin pour les peaux grasses

| FORMULE | | |
|---|---|---|
| A | MICROPEARL™ M310 | 1,0% |
| | Composition 1 | 5,0% |
| | Isononanoate d'octyle | 4.0% |
| B | eau | q.s.p.100% |
| C | SEPICONTROL™ A5 | 4,0% |
| | Parfum | 0,1% |
| | SEPICIDE™ HB | 0,3% |
| | SEPICIDE™ CI | 0,2% |
| D | CAPIGEL™ 98 | 0,5% |
| | Eau | 10% |

### Exemple 27 : Crème aux AHA

| FORMULE | | |
|---|---|---|
| A | MONTANOV™ 68 | 5,0% |
| | LIPACIDE™ PVB | 1,05% |
| | LANOL™ 99 | 10,0% |
| B | eau | q.s.p.100% |
| | Acide gluconique | 1,5% |
| | TEA (triéthanolamine) | 0,9% |
| C | Composition 2 | 1,5% |
| D | parfum | 0,4% |
| | SEPICIDE™ HB | 0,2% |
| | SEPICIDE™ Cl | 0,4% |

### Exemple 28 : Autobronzant non gras pour visage et corps

| FORMULE | | |
|---|---|---|
| A | LANOL™ 2681 | 3,0% |
| | Composition 1 | 2,5% |
| B | eau | q.s.p.100% |
| | Dihydroxyacétone | 3,0% |
| C | parfum | 0,2% |
| | SEPICIDE™ HB | 0,8% |
| | NaOH (hydroxyde de sodium) | qs pH = 5 |

### Exemple 29 : Lait solaire au monoï de Tahiti

| FORMULE | | |
|---|---|---|
| A | Monoï de Tahiti | 10% |
| | LIPACIDE™ PVB | 0,5% |
| | Composition 2 | 2,2% |
| B | eau | q.s.p.100% |
| C | parfum | 0,1% |
| | SEPICIDE™ HB | 0,3% |
| | SEPICIDE™ CI | 0,1% |
| | PARSOL™ MCX | 4,0% |

### Exemple 30 : Soin solaire pour le visage

| FORMULE | | |
|---|---|---|
| A | Cyclométhicone et diméthiconol | 4,0% |
| | Composition 2 | 3,5% |
| B | eau | q.s.p.100% |
| C | parfum | 0,1% |
| | SEPICIDE™ HB | 0,3% |
| | SEPICIDE™ CI | 0,21% |
| | PARSOL™ MCX | 5,0% |
| | Micatitane | 2,0% |
| | Acide lactique | q.s.p. pH = 6,5 |

### Exemple 31 : Emulsion bronzante sans soleil

| FORMULE | | |
|---|---|---|
| A | LANOL™ 99 | 15% |
| | MONTANOV™ 68 | 5,0% |
| | PARSOL™ MCX | 3,0% |
| B | eau | q.s.p.100% |
| | Dihydroxyacétone | 5,0% |
| | Phosphate monosodique | 0,2% |
| C | Composition 1 | 0,5% |
| D | parfum | 0,3% |
| | SEPICIDE™ HB | 0,8% |
| | NaOH | q.s. pH=5. |

Les caractéristiques des produits utilisés dans les exemples précédents, sont les suivantes :
Le MONTANOV™ 68 (cétéaryl glucoside, alcool cétéarylique), est une composition auto-émulsionnable telle que celles décrites dans WO 92/06778, commercialisée par la société SEPPIC.
Le MONTANOV™ 202 (arachidyl glucoside, alcool arachydilique + alcool béhènylique), est une composition auto-émulsionnable telle que celles décrites dans WO 98/17610, commercialisée par la société SEPPIC.
Le MICROPEARL™ M 305 est une poudre hydrodispersible soyeuse à base de copolymer méthylméthacrylate réticulé.
Le MICROPEARL™ M 100 est une poudre ultra fine au toucher très doux et à action matifiante commercialisée par la société MATSUMO
Le SEPICIDE™ Cl, imidazoline urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL ™ S sont des facteurs de consistance commercialisés par la société SEPPIC.
Le SEPICIDE™ HB , qui est un mélange de phénoxyéthanol, de méthylparaben, d'éthylparaben, de propylparaben et de butylparaben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le SEPIPERL™ N est un agent nacrant, commercialisé par la société SEPPIC, à base d'un mélange d'alkyl polyglucosides tels que ceux décrits dans WO 95/13863.
Le MONTANOV™ S est un agent nacrant, commercialisé par la société SEPPIC, à base d'un mélange d'alkyl polyglucosides tels que ceux décrits dans WO 95/13863.
Le PECOSIL™ PS100 est du diméthicone copolyol phosphate commercialisé par la société PHOENIX.
Le LANOL™ 99 est de l'isononyl isononanoate commercialisé par la société SEPPIC.
Le LANOL™ 37T est du triheptanoate de glycérol, commercialisé par la société SEPPIC.
Le SEPIFEEL™ ONE est un mélange de palmitoylproline, de palmitoyl glutamate de magnésium et de palmitoyl sarcosinate de magnésium, tel que ceux décrits dans FR 2787323.
Le SOLAGUM™ L est un carraghénane commercialisé par la société SEPPIC.
Le MARCOL™ 82 est une huile de paraffine commercialisée par la société ESSO.
Le LANOL™ 84D est du malate de dioctyle commercialisé par la société SEPPIC.
Le PARSOL™ MCX est du paraméthoxycinnamate d'(éthyl hexyle) commercialisé par la société GIVAUDAN.
I' EUSOLEX™ 4360 est de la benzophénone-3 commercialisé par la société MERCK.
Le DOW CORNING™ 245 Fluid est de la cyclométhicone, commercialisée par la société DOW CORNING.
Le LIPACIDE™ PVB,est un hydrolysat de protéines de blé palmitoylé, commercialisée par la société SEPPIC.
Le SEPICONTROL™ A5 est un mélange capryloyl glycine, sarcosine, extrait de cinnamon zylanicum, commercialisé par la société SEPPIC, tel que ceux décrits dans la demande internationale de brevet PCT/FR98/01313 déposée le 23 juin 1998.
Le CAPIGEL™ 98 est un copolymère d'acrylates commercialisé par la société SEPPIC.
Le LANOL™ 2681 est un mélange caprylate, caprate de coprah, commercialisé par la société SEPPIC.

## Revendications

1. Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, **caractérisé en ce que** ledit polyélectrolyte est, soit un homopolymère à base d'un monomère possédant ou bien une fonction acide fort partiellement ou totalement salifiée, ou bien une fonction acide faible partiellement ou totalement salifiée, soit un copolymère à base d'au moins un monomère possèdant une fonction acide fort, copolymérisé ou bien avec au moins un monomère possédant une fonction acide faible, ou bien avec au moins un monomère neutre, soit un copolymère à base d'au moins un monomère possédant une fonction acide faible, copolymérisé avec au moins un monomère neutre et **caractérisé en ce que** le solvant constitutif de la phase huile est choisi parmi les esters d'acides gras.

2. Composition telle que définie à la revendication 1 dans laquelle le solvant constitutif de la phase huile est choisi parmi les composés de formule (I) :
R₁-(C=O)-O-[[CH₂-CH[O-[C(=O)]ₘ-R₂]-CH₂-O]ₙ-[C(=O)]ₚ]_{q}-R₃ (I)
dans laquelle :
R₁ représente une chaîne hydrocarbonée saturée ou insaturée, linéaire ou ramifiée comportant de 7 à 30 atomes de carbone,
R₂ représente indépendamment de R₁, un atome d'hydrogène, une chaîne hydrocarbonée saturée ou insaturée linéaire ou ramifiée comportant de 7 à 30 atomes de carbone,
R₃ représente indépendamment de R₁ ou de R₂, un atome d'hydrogène ou une chaîne hydrocarbonée saturée ou insaturée, linéaire ou ramifiée comportant de 1 à 30 atomes de carbone,
m, n, p et q sont indépendamment l'un de l'autre égaux à 0 ou à 1, étant entendu que lorsque R₃ représente un atome d'hydrogène, q est différent de 0.

3. Composition telle que définie à la revendication 2, pour laquelle dans la formule (I), R₁, R₂ et R₃ représentent, indépendamment les uns des autres, un radical choisi parmi les radicaux heptyle, octyle, nonyle, décyle, undécyle, dodécyle, tridécyle, tétradécyle, pentadécyle, hexadécyle, heptadécyle, octadécyle, nonadécyle, eicosyle, heneicosyle, docosyle, heptadécènyle, eicosènyle, heneicosènyle, docosènyle, heptadécadiènyle ou décènyle.

4. Composition telle que définie à la revendication 3, pour laquelle dans la formule (I), le groupe R₁-C(=O)- représente l'un des radicaux octanoyle (caprylyle), décanoyle, undécylènoyle, dodécanoyle (lauroyle), tétradécanoyle (myristyle), hexadécanoyle (palmitoyle), octadécanoyle (stéaryle), eicosanoyle (arachidoyle), docosanoyle (béhènoyle), 8-octadécènoyle (oléyle), éicosènoyle (gadoleoyle), 13-docosènoyle (érucyle), 9,12-octadécadiènoyle (linoléoyle) ou 9,12,15-octadécatriénoyle (linolénoyle).

5. Composition telle que définie à l'une quelconques des revendications 2 à 4, pour laquelle le solvant constitutif de la phase huile du latex inverse, est un composé de formule (la) :
R₁-(C=O)-O-CH₂-CH[O-[C(=O)]ₘ-R₂]-CH₂-O-[C(=O)]ₚ-R₃ (Ia)
correspondant à la formule (I), dans laquelle q et n sont égaux à 1, ou un mélange de composés de formule (la).

6. Composition telle que définie à la revendication 5 pour laquelle le solvant constitutif de la phase huile du latex inverse, est un composé de formule (la₁) :
R₁-(C=O)-O-CH₂-CH(OH)-CH₂-OH (la₁)
correspondant à la formule (la) dans laquelle m et p sont égaux à 0 et R₂ et R₃ représentent un atome d'hydrogène.

7. Composition telle que définie à la revendication 5 pour laquelle le solvant constitutif de la phase huile du latex inverse, est un composé de formule(la₂) :
R₁-(C=O)-O-CH₂-CH(OH)-CH₂-O-C(=O)-R₃ (la₂)
correspondant à la formule (la) dans laquelle p est égal à 1, m est égal à 0 et R₂ représente un atome d'hydrogène.

8. Composition telle que définie à la revendication 5 pour laquelle le solvant constitutif de la phase huile du latex inverse, est un composé de formule (la₃) :
R₁-(C=O)-O-CH₂-CH[O-C(=O)-R₂]-CH₂-O-C(=O)-R₃ (la₃)
correspondant à la formule (la) dans laquelle m et p sont égaux à 1.

9. Composition telle que définie aux revendications 5 à 8, pour laquelle le solvant constitutif de la phase huile du latex inverse, est un mélange de composés de formules (la₁), (la₂) et/ou (la₃).

10. Composition telle que définie à l'une quelconques des revendications 2 à 4, pour laquelle le solvant constitutif de la phase huile du latex inverse, est un composé de formule (lb) :
R₁-(C=O)-O-R₃ (Ib)
correspondant à la formule (I) dans laquelle q est égal à 0, ou un mélange de composés de formules (lb).

11. Composition telle que définie à la revendication 10, pour laquelle le solvant constitutif de la phase huile du latex inverse est le palmitate d'octyle.

12. Composition telle que définie aux revendications 5 à 11, pour laquelle le solvant constitutif de la phase huile du latex inverse, est un mélange d'au moins un composé de formule (lb) et d'au moins un composé de formule (la).

13. Composition telle que définie à l'une quelconques des revendications 1 à 12, dans laquelle le ou les agents émulsifiants du type eau dans huile, sont choisis parmi le monooléate de sorbitan, l'isostéarate de sorbitan ou l'oléate de sorbitan éthoxylé avec 5 moles d'oxyde d'éthylène.

14. Composition telle que définie à l'une quelconques des revendications 1 à 13, dans laquelle le ou les agents émulsifiants du type huile dans eau sont choisis parmi l'oléate de sorbitan éthoxylé avec 20 moles d'oxyde d'éthylène, l'huile de ricin éthoxylée à 40 moles d'oxyde d'éthylène, le laurate de sorbitan éthoxylé à 20 moles d'oxyde d'éthylène, l'alcool laurique éthoxylé à 7 moles d'oxyde d'éthylène.

15. Composition telle que définie à l'une quelconques des revendications 1 à 14, dans laquelle le ou les agents émulsifiants du type huile dans eau sont choisis parmi les composés de formule (II) :
R₄-O-[CH(R₅)-CH₂-O]ₙ-(G)ₓ-H (II)
dans laquelle R₄ représente un radical hydrocarboné linéaire ou ramifié, saturé ou insaturé comprenant de 1 à 30 atomes de carbones, R₅ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30.

16. Composition telle que définie à la revendication 15, pour laquelle dans la formule (II), x est compris entre 1 et 3, plus particulièrement entre 1,05 et 2,5, tout particulièrement entre 1,1 et 2,0 et de préférence, inférieur ou égal à 1,5.

17. Composition telle que définie à l'une des revendications 15 ou 16, pour laquelle, dans la formule (II), G représente le reste du glucose ou le reste du xylose et n est égal à 0.

18. Composition telle que définie à l'une quelconque des revendications 15 à 17, pour laquelle, dans la formule (II), R₄ représente un radical comportant de 8 à 18 atomes de carbone et plus particulièrement un radical octyle, décyle, undécyle, dodécyle, tétradécyle ou hexadécyle, lesdits radicaux étant linéaires ou ramifiés.

19. Composition telle que définie à l'une quelconque des revendications 1 à 18, pour laquelle la fonction acide fort du monomère en comportant, est la fonction acide sulfonique ou la fonction acide phosphonique partiellement ou totalement salifiée et de préférence le monomère est l'acide 2-méthyl 2-[(1-oxo 2-propènyl) amino] 1-propanesulfonique partiellement ou totalement salifiée sous forme d'un sel de métal alcalin tel que par exemple le sel de sodium ou le sel de potassium, de sel d'ammonium, de sel d'un amino alcool tel que par exemple le sel de monéthanolamine ou de sel d'un amino acide tel que par exemple le sel de lysine.

20. Composition telle que définie à l'une quelconque des revendications 1 à 19, pour laquelle la fonction acide faible du monomère en comportant est la fonction acide carboxylique et de préférence, ledit monomère est choisi parmi l'acide acrylique, l'acide méthacrylique, l'acide itaconique ou l'acide maléique partiellement ou totalement salifié.

21. Composition telle que définie à l'une quelconque des revendications 1 à 20 pour laquelle le monomère neutre est choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters.

22. Composition telle que définie à l'une des revendications 1 à 18, dans laquelle le polyélectrolyte est un homopolymère d'acide acrylique partiellement ou totalement salifié sous forme de sel de sodium ou de sel d'ammonium.

23. Composition telle que définie à l'une des revendications 1 à 18, dans laquelle le polyélectrolyte est un copolymère d'acide 2-méthyl 2-[(1-oxo 2-propènyl) amino] 1-propanesulfonique partiellement ou totalement salifié (a), et d'acrylate de (2-hydroxy éthyle) (b), dans un rapport molaire (a) / (b) compris entre 30 / 70 et 90 / 10 et tout particulièrement 50 / 50 et 90 / 10.

24. Composition telle que définie à la revendication 23, dans laquelle le polyélectrolyte est un copolymère de sel de sodium ou de sel d'ammonium de l'acide 2-méthyl 2-[(1-oxo 2-propènyl) amino] 1-propanesulfonique (a₁) et de 10% à 40% d'acrylate de (2-hydroxy éthyle) (b), dans un rapport molaire (a₁) / (b), compris entre 60 / 40 et 90 / 10.

25. Composition telle que définie à l'une des revendications 1 à 18, dans laquelle le polyélectrolyte est un copolymère de sel de sodium de sel d'ammonium, de sel de monoéthanolamine ou de sel de lysine de l'acide 2-méthyl 2-[(1-oxo 2-propènyl) amino] 1-propanesulfonique (a₁) et d'acide acrylique partiellement ou totalement salifié sous forme de sel de sodium, de sel d'ammonium, de sel de monoéthanolamine ou de sel de lysine (c₁), dans un rapport molaire (a₁) / (c₁), compris entre 30 / 70 et 90/10 et tout particulièrement entre 30 / 70 et 45 / 55.

26. Composition telle que définie à l'une des revendications 1 à 18, dans laquelle le polyélectrolyte est un copolymère de sel de sodium ou de sel d'ammonium de l'acide 2-méthyl 2-[(1-oxo 2-propènyl) amino] 1-propanesulfonique (a₂) et d'acrylamide (d), dans un rapport molaire (a₂) / (d), compris entre 50 / 50 et 30/70.

27. Composition telle que définie à l'une quelconque des revendications 1 à 26, **caractérisée en ce que** le polyélectrolyte est réticulé et/ou branché avec un composé diéthylénique ou polyéthylénique dans la proportion molaire exprimée par rapport aux monomères mis en oeuvre, de 0,005% à 1%, et de préférence de 0,01 % à 0,5 % et, plus particulièrement de 0,1 % à 0,25 %.

28. Composition telle que définie à la revendication 27, **caractérisée en ce que** l'agent de réticulation et/ou de ramification est choisi parmi l'acide diallyoxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène bis(acrylamide), le triallylamine, ou un mélange de ces composés.

29. Composition telle que définie à l'une quelconque des revendications 1 à 28, **caractérisée en ce qu'**elle contient de 4% à 10% en poids, d'agents émulsifiants.

30. Composition telle que définie à la revendication 29, dans laquelle de 20% à 50% et plus particulièrement de 25% à 40% du poids total des émulsifiants sont du type eau dans huile et de 80% à 50% et plus particulièrement de 75 à 60% en poids sont du type huile dans eau.

31. Composition telle que définie à l'une quelconque des revendications 1 à 30, **caractérisée en ce que** la phase huile représente de 15% à 40%, et de préférence de 20% à 25%, de son poids total.

32. Composition telle que définie à l'une quelconque des revendications 1 à 31, **caractérisée en ce qu'**elle comporte en outre, un ou plusieurs additifs choisis parmi les agents complexant, les agents de transfert ou les agents limiteurs de chaînes.

33. Composition cosmétique, dermocosmétique, dermopharmaceutique ou pharmaceutique, **caractérisée en ce qu'**elle comprend de 0,1 % à 10 % en poids, de la composition telle que définie à l'une quelconque des revendications 1 à 32.

34. Composition cosmétique, dermocosmétique, dermopharmaceutique ou pharmaceutique telle que définie à la revendication 33, sous la forme d'un lait, d'une lotion, d'un gel, d'une crème, d'un savon, d'un bain moussant, d'un baume, d'un shampooing ou d'un après shampooing.

35. Utilisation d'une composition telle que définie à l'une des revendications 1 à 34, pour préparer des compositions cosmétiques, dermocosmétiques, dermopharmaceutiques ou pharmaceutiques.
